# EUROPEAN PATENT APPLICATION

(11) **EP 1 688 494 A1**
(43) Date of publication of application: **09.08.2006**
(21) Application number: 04799448.8
(22) Date of filing: 01.11.2004
(51) Int. Cl.: C12N 15/12, C07K 14/705, C12N 1/15, C12N 1/19, C12N 1/21, C12N 5/00, C12Q 1/02, G01N 33/15

(54) **MONKEY NPY Y4 RECEPOTR AND METHOD OF EVALUATING COMPOUND**

(30) Priority: 30.10.2003 JP 2003369840
(71) Applicant: BANYU PHARMACEUTICAL CO., LTD., Tokyo 102-8667 (JP)
(72) Inventor: SANO, Hideki, c/o Banyu Pharmaceutical Co.,Ltd., Tsukuba-shi, Ibaraki 3002611 (JP); IWAASA, Hisashi, c/o Banyu Pharmaceutical Co.,Ltd., Tsukuba-shi, Ibaraki 3002611 (JP)
(74) Representative: Rollins, Anthony John
(86) International application number: PCT/JP2004/016242
(87) International publication number: WO 2005/042742

(57) **Abstract**

There are provided a non-human primate NPY Y4 gene etc., using a nucleic acid listed as SEQ ID NO: 1 or 3, a protein listed as SEQ ID NO: 2 or 4, a recombinant vector containing a gene consisting of the nucleic acid, and a transformant cell containing the recombinant vector, whereby it is possible to evaluate and screen for compounds that act on the NPY Y4 receptor.

## Description

### Technical Field

The present invention relates to a novel simian Neuropeptide-Y (hereinafter abbreviated as NPY) Y4 receptor gene and protein, a recombinant vector containing the gene, a transformant containing the recombinant vector and a compound evaluation method using the gene or protein.

### Background Art

NPY is a peptide composed of 36 amino acids which was isolated from swine brain in 1982 by Tatemoto et al. (Non-patent document 1). NPY is distributed widely throughout the central and peripheral nervous systems and as one of the most abundant nervous system peptides it carries out a variety of functions in the body.

Specifically, NPY acts in the central nervous system as an appetite promoting substance while also notably accelerating fat accumulation via secretion of various hormones or actions of the nervous system. For example, continuous intraventricular administration of NPY is known to induce obesity and insulin resistance, based on the actions mentioned above. Moreover, NPY is also known to have central actions such as depression, anxiety, schizophrenia, pain, dementia and circadian rhythm regulation. In the periphery, NPY is found with norepinephrine in the sympathetic nerve endings and is involved in sympathetic nervous system tension. Peripheral administration of NPY is known to cause vasoconstriction, and to reinforce the action of other vasoconstrictive substances such as norepinephrine.

NPY exhibits a myriad of pharmacological actions via its receptors, some of which it shares in common with its analogs Peptide YY (hereinafter abbreviated as PYY) and Pancreatic Polypeptide (hereinafter abbreviated as PP). It is known that the pharmacological actions of NPY are induced via at least five different receptor classes NPY Y1 to Y5, either alone or interactively (Non-patent document 2). Among these, NPY Y4 receptor has high affinity for PP, and its particularly notable pharmacological actions that have been reported include suppression of pancreatic exocrine secretion and enterokinesis (Non-patent document 3). In the central nervous system, it is known to accelerate secretion of sex hormones (Non-patent document 4). In addition, it has been discovered that appetite is suppressed by peripheral administration of PP to mice or humans (Non-patent document 5, Non-patent document 6).

The function of NPY and its related peptides is exhibited upon binding to NPY receptors in the central and peripheral nervous system. Thus, inhibition of binding between NPY or its related peptides with NPY receptors can block expression of their pharmacological action. As a result, substances that antagonize binding between NPY or its related peptides and NPY receptors are expected to be useful for prevention or treatment of various conditions associated with NPY and its related peptides, e.g. circulatory system disorders such as angina pectoris, acute/congestive heart failure, myocardial infarction, hypertension, kidney disease, electrolyte imbalance and vasospasm, nervous system disorders such as bulimia, depression, anxiety, convulsion, epilepsy, dementia, pain, alcoholism, drug withdrawal symptoms, circadian rhythm alteration, schizophrenia, dysmnesia, sleep disorder and cognitive impairment, metabolic disorders such as obesity, diabetes, hormone secretion imbalances, gout and fatty liver, and reproductive disorders such as infertility, premature birth and sexual dysfunction, as well as gastrointestinal diseases, respiratory diseases, inflammatory conditions and glaucoma. Recently, certain types of NPY receptor antagonists have been discovered which are useful for prevention or treatment of hypercholesterolemia, hyperlipidemia and arteriosclerosis (Patent document 1).

Thus, since elucidation of the biological functioning of NPY, its related peptides and their receptors is essential for development of therapeutic and diagnostic agents for the various conditions mentioned above, rapid advances in analysis have been pursued on the genetic level. Genetic analysis of the NPY Y4 receptor has already led to isolation of the human NPY Y4 receptor gene (Non-patent document 7, Non-patent document 8, Patent document 2). The human NPY Y4 receptor was isolated by screening a human genome library using NPY Y1 receptor cDNA as a probe, and it was established as having a seven membrane-spanning structure with 42% homology to Y1 receptor on the amino acid level. It was also demonstrated to have high affinity for PP as well as NPY, and similar to NPY Y1 receptor and Y2 receptor, to increase intracellular calcium concentration and exhibit an inhibitory effect on cAMP production.

The physiological actions of candidate compounds developed for therapeutic or diagnostic agents are evaluated in rodents and primates. This is because candidate compounds often exhibit different drug effects depending on the animal species, and evaluation in primates, which are more closely related to humans, can lead to development of more effective therapeutic and diagnostic agents.
Patent document 1: International Publication Pamphlet WO 99/27965
Patent document 2: International Publication Pamphlet WO 95/17906

Non-patent document 1: Nature, Vol. 296, 659 (1982)
Non-patent document 2: Trends in Neuroscience, Vol. 20, 294 (1997)
Non-patent document 3: Gastroenterology, Vol. 85, 1411 (1983)
Non-patent document 4: Endocrinology, Vol. 140, 5171 (1999)
Non-patent document 5: Gastroenterology, Vol. 124, 1325 (2003)
Non-patent document 6: The Journal of Clinical Endocrinology & Metabolism, Vol. 88, 3989 (2003)
Non-patent document 7: The Journal of Biological Chemistry, Vol. 270, 26762 (1995)
Non-patent document 8: The Journal of Biological Chemistry, Vol. 270, 29123 (1995)

### Disclosure of the Invention

### Problem to be Solved by the Invention

Nevertheless, the NPY Y4 receptor genes of primates other than humans have not yet been isolated, and therefore evaluation of therapeutic or diagnostic agents using the genes is still not possible.

It is an object of the present invention, which has been accomplished in light of the aforementioned problems of the prior art, to provide the NPY Y4 receptor gene and protein of a non-human primate. It is another object of the invention to provide a compound evaluation method using the gene and protein.

### Means for Solving the Problem

As a result of much diligent research directed toward achieving the objects stated above, the present inventors succeeded in isolating the rhesus monkey NPY Y4 receptor gene and evaluating ligands by binding assays using the receptor, and have thereupon completed this invention.

Specifically, a nucleic acid of the invention is characterized by containing the nucleotide sequence listed as SEQ ID NO: 1 or 3.

A nucleic acid of the invention is also characterized by consisting of the nucleotide sequence listed as SEQ ID NO: 1 or 3. A simian NPY Y4 receptor gene (simian gene coding for a protein having NPY Y4 receptor activity) consisting of this nucleic acid is also provided according to the invention.

A nucleic acid of the invention is further characterized by coding for a protein consisting of the amino acid sequence listed as SEQ ID NO: 2 or 4. A simian NPY Y4 receptor gene consisting of this nucleic acid is also provided according to the invention.

A nucleic acid of the invention is further characterized by being a simian isolated nucleic acid which hybridizes under stringent conditions with a nucleic acid consisting of the nucleotide sequence listed as SEQ ID NO: 1 or 3 or a nucleotide sequence complementary thereto and which codes for a protein having NPY Y4 receptor activity. A simian NPY Y4 receptor gene consisting of this nucleic acid is also provided according to the invention.

A recombinant vector of the invention is characterized by comprising the aforementioned nucleic acid or gene. A transformant cell of the invention is characterized by comprising the aforementioned recombinant vector.

A protein of the invention is characterized by containing the amino acid sequence listed as SEQ ID NO: 2 or 4.

A protein of the invention is also characterized by consisting of the amino acid sequence listed as SEQ ID NO: 2 or 4.

A protein of the invention is further characterized by being an isolated protein consisting of the amino acid sequence listed as SEQ ID NO: 2 or 4 with a substitution, deletion, addition or insertion of one or more amino acids, and having NPY Y4 receptor activity.

By utilizing such a nucleic acid, gene, protein, recombinant vector or transformant cell it is possible to construct an expression system for a primate (mainly simian) NPY Y4 receptor or a mutant thereof, and to construct a compound evaluation system using the expression system.

A compound evaluation method of the invention is characterized by comprising a step of transferring a simian NPY Y4 receptor gene into a cell to prepare a cell expressing the receptor, a step of contacting a test compound with the cell, and a step of detecting specific binding of the test compound to the receptor.

A compound evaluation method of the invention is further characterized by comprising a step of transferring a simian NPY Y4 receptor gene into a cell to prepare a cell expressing the receptor, a step of contacting a test compound with the cell, a step of assaying the activity of an intracellular signal transducer produced by the contact, and a step of comparing the activity with the activity of the intracellular signal transducer without contact with the test compound.

A compound evaluation method of the invention is still further characterized by comprising a step of contacting a test compound with a simian NPY Y4 receptor protein (simian protein having NPY Y4 receptor activity) and a step of detecting a change in activity of the protein caused by the contact.

These compound evaluation methods allow evaluation and screening of compounds which are candidates for therapeutic and diagnostic agents using a primate NPY Y4 receptor, thereby permitting development of effective drugs in a model system more closely resembling that of humans.

### Effects of the Invention

With the nucleic acid, protein and compound evaluation method of the invention, it is possible to obtain an NPY Y4 receptor gene and protein of a non-human primate, and to evaluate and screen compounds using the gene or protein. The gene or protein may therefore be used for evaluation and development of therapeutic and diagnostic agents.

### Brief Description of the Drawings

Fig. 1 is a graph showing the relationship between membrane fraction concentration and human PP binding when using a nucleic acid (SEQ ID NO: 1) of the invention.
Fig. 2 is a graph showing the relationship between membrane fraction concentration and human PP binding when using a nucleic acid (SEQ ID NO: 3) of the invention.
Fig. 3 is a graph showing the relationship between human PP, human NPY and swine PYY peptide concentrations and human PP binding when using a nucleic acid (SEQ ID NO: 1) of the invention.
Fig. 4 is a graph showing the relationship between human PP, human NPY and swine PYY peptide concentrations and human PP binding when using a nucleic acid (SEQ ID NO: 3) of the invention.
Fig. 5 is a graph showing the relationship between human PP concentration and fluorescence intensity changes in cells transfected with a nucleic acid (SEQ ID NO: 1 or 3) of the invention.

### Best Modes for Carrying Out the Invention

Preferred embodiments of the invention will now be explained in detail.

"Nucleic acid" according to the invention refers to, for example, DNA, RNA or modified DNA or RNA, and preferably it is DNA. The DNA may be either genomic DNA or cDNA, and either single-stranded or double-stranded.

An "isolated" nucleic acid or protein according to the invention refers to nucleic acid or protein which is substantially free of cellular substances and culturing medium when produced by a recombinant DNA technique, or substantially free of precursor substances or other substances when produced by chemical synthesis.

The phrase "hybridizes under stringent conditions" according to the invention means that two nucleic acid fragments hybridize with each other under the hybridization conditions described in Molecular Cloning: A Laboratory Manual, 2nd Edition, Cold Spring Harbor (1989), 9.47-9.62 and 11.45-11.61. More specifically, there may be mentioned conditions wherein, for example, hybridization is conducted at about 45°C, 6.0 x SSC and followed by rinsing at 50°C, 2.0 x SSC. For selection of stringency, the salt concentration for the rinsing step may be selected from about 2.0 x SSC, 50°C for low stringency to about 0.2 x SSC, 50°C for high stringency. The temperature for the rinsing step may be from room temperature (about 22°C) for low stringency conditions to about 65°C for high stringency conditions.

A rhesus monkey NPY Y4 receptor according to the invention will now be explained.

The nucleotide sequences of two of the rhesus monkey NPY Y4 receptor genes of the invention have been determined (SEQ ID NO: 1 and 3), and their sequences differ in a single nucleotide. These genes each have an open reading frame of 1128 nucleotides, coding for a protein of 375 amino acids (SEQ ID NO: 2 and 4). However, the 1111th nucleotide in the coding region differs, resulting in a difference in the 361st amino acid. The present inventors assume that this difference is a genetic polymorphism.

There are no particular restrictions on the method of cloning the rhesus monkey NPY Y4 receptor gene, and as a specific example there may be mentioned a method wherein a nucleic acid fragment from the rhesus monkey NPY Y4 receptor gene or a gene having high homology thereto is used for amplification of the full length cDNA by 5'-RACE or 3'-RACE, or a method wherein a cDNA library constructed using an appropriate vector (plasmid vector, bacteriophage, etc.) is screened using a nucleic acid fragment from the rhesus monkey NPY Y4 receptor gene. These methods may be carried out according to protocols that are well known to those skilled in the art, with reference to Proc. Natl. Acad. Sci. USA, Vol. 85, 8998 (1998), for example.

The homology between the rhesus monkey NPY Y4 receptor gene listed as SEQ ID NO: 1 and the human NPY Y4 receptor gene (GenBank Accession No. NM_005972) is approximately 95.6% in the coding region, with a difference of 50 nucleotides. The homology between the rhesus monkey NPY Y4 receptor gene listed as SEQ ID NO: 3 and the human NPY Y4 receptor gene is approximately 95.5% in the coding region, with a difference of 51 nucleotides. Nucleic acids of the invention include a nucleic acid with the nucleotide sequence of SEQ ID NO: 1 or 3 having a substitution, deletion, insertion or addition of one or more nucleotides, and there may be a substitution, deletion, insertion or addition of preferably 1-49 nucleotides, more preferably 1-30 nucleotides, even more preferably 1-20 nucleotides, still more preferably 1-10 nucleotides and yet still more preferably 1-5 nucleotides. A nucleotide sequence of the nucleic acid having a substitution, deletion, insertion or addition preferably has at least 96% homology, more preferably at least 97% homology, even more preferably at least 98% homology and most preferably at least 99% homology to the nucleotide sequence listed as SEQ ID NO: 1 or 3. The nucleic acid with a substitution, deletion, insertion or addition may be obtained by isolating a nucleic acid of the invention which hybridizes under stringent conditions with a nucleic acid consisting of the nucleotide sequence listed as SEQ ID NO: 1 or 3 or a nucleotide sequence that is complementary thereto, and so long as the nucleic acid is biologically active there are no particular restrictions on the species, although specifically a simian NPY Y4 receptor gene (gene coding for a protein with NPY Y4 receptor activity) is preferred, with examples of simian NPY Y4 receptor genes including NPY Y4 receptor genes of rhesus monkey, cynomolgus monkey, Japanese monkey, squirrel monkey, green monkey, anubis baboon and common marmoset.

The present invention also encompasses a protein having the amino acid sequence listed as SEQ ID NO: 2 or 4, as well as a mutant thereof. A protein having the amino acid sequence listed as SEQ ID NO: 2 or 4 consists of 375 amino acids, as mentioned above. These proteins differ in the 361st amino acid. As mutants there may be mentioned proteins consisting of the amino acid sequence listed as SEQ ID NO: 2 or 4 with a substitution, deletion or insertion of one or between 2 and 16 amino acids, and proteins consisting of the same amino acid sequence with an addition of one or more amino acids. Such mutant proteins are preferably biologically active, and more preferably have NPY Y4 receptor activity.

The method of preparing the rhesus monkey NPY Y4 receptor protein is not particularly restricted, and specifically there may be mentioned a method wherein an isolated rhesus monkey NPY Y4 receptor gene is inserted into an expression vector, the expression vector (recombinant vector) is transferred into host cells such as animal cells, insect cells or *E. coli* cells for expression of the gene, and the protein (gene product) is purified. Such methods may be conducted by protocols that are well known to those skilled in the art, and for example, they may be conducted with reference to Molecular Cloning: A Laboratory Manual, 2nd and 3rd Editions, Cold Spring Harbor (1989, 2001).

The invention further provides an expression vector (recombinant vector) containing any one of the following nucleic acids or genes.
1. An isolated nucleic acid comprising the nucleotide sequence listed as SEQ ID NO: 1 or 3.
2. An isolated nucleic acid consisting of the nucleotide sequence listed as SEQ ID NO: 1 or 3, or a simian NPY Y4 receptor gene consisting of this nucleic acid.
3. An isolated nucleic acid coding for a protein consisting of the amino acid sequence listed as SEQ ID NO: 2 or 4, or a simian NPY Y4 receptor gene consisting of this nucleic acid.
4. A simian isolated nucleic acid which hybridizes under stringent conditions with a nucleic acid consisting of the nucleotide sequence listed as SEQ ID NO: 1 or 3 or a nucleotide sequence complementary thereto and which codes for a protein having NPY Y4 receptor activity, or a simian NPY Y4 receptor gene consisting of this nucleic acid.

The recombinant vector of the invention may be obtained by inserting the nucleic acid or gene of the invention into an appropriate expression vector by a publicly known method. The expression vector used is not particularly restricted so long as it is well known to those skilled in the art, and as examples there may be mentioned pcDNA3.1, pBlueBacHis2, pCI-neo, pcDNAI, pMC1neo, pXT1, pSG5, pEF1/V5-HisB, pCR2.1, pET1 and λgt11. The recombinant vector of the invention is preferably one that is capable of autonomous replication in host cells, and contains a promoter, a ribosome binding sequence (SD sequence) and a terminator.

The phrase "comprising the nucleotide sequence listed as SEQ ID NO: 1 or 3" in 1. above means that there may be added to the aforementioned nucleotide sequence a vector linker sequence, or a sequence necessary for modification of the gene, such as a restriction endonuclease cleavage site used for gene recombination.

The invention further provides a host cell (transformant cell) containing the aforementioned recombinant vector.

The transformant cell of the invention may be obtained by transferring the recombinant vector of the invention into suitable host cells by a publicly known method. The host cell used is not particularly restricted so long as it is known to those skilled in the art, and may be an animal cell, insect cell, plant cell or microbe. Specifically, as examples there may be mentioned COS1, COS7, CHO, NIH/3T3, 293, Raji, CV11, C1271, MRC-5, CPAE, L-M(TK-), HeLa and Sf9. The method of transferring the recombinant vector into host cells is not particularly restricted so long as it is a publicly known method, and specifically there may be mentioned electroporation, the calcium phosphate method, the DEAE-dextran method, lipofection and the gene gun method.

The invention also encompasses an antibody for the protein having the amino acid sequence listed as SEQ ID NO: 2 or 4 and a mutant thereof. The antibody may be prepared using the protein or a partial peptide thereof as an antigen, and may be monoclonal or polyclonal antibody. The antibody may react not only with simian NPY Y4 receptor proteins but also with NPY Y4 receptor proteins of other species depending on the amino acid sequence of the epitope, but for preparation of an antibody that is selective only for simian NPY Y4 receptor proteins, an antibody with the desired specificity can be prepared by creating a monoclonal antibody using as a peptide antigen a region of a simian NPY Y4 receptor protein which has low homology to NPY Y4 receptor proteins of other species.

Uses of the nucleic acid having the nucleotide sequence listed as SEQ ID NO: 1 or 3 and the protein having the amino acid sequence listed as SEQ ID NO: 2 or 4 according to the invention will now be explained.

### (1) Evaluation of compounds

The nucleic acid or protein of the invention may be used to evaluate compounds that act on the NPY Y4 receptor. The method of detecting the presence and level of action on the NPY Y4 receptor may be a method wherein specific binding of a test compound to the receptor is detected, a method wherein a gene expression level which is altered by contact with the test compound is detected, or a method wherein the activity of intracellular signal transduction via the NPY Y4 receptor produced by the contact is detected. This will be explained below.

A compound evaluation method of the invention is characterized by comprising a step of transferring a simian NPY Y4 receptor gene into a cell to prepare a cell expressing the receptor, a step of contacting a test compound with the cell, and a step of detecting specific binding of the test compound to the receptor.

A second compound evaluation method of the invention is characterized by comprising a step of transferring a simian NPY Y4 receptor gene into a cell to prepare a cell expressing the receptor, a step of contacting a test compound with the cell, a step of assaying the activity of an intracellular signal transducer produced by the contact, and a step of comparing the activity with the activity of the intracellular signal transducer without contact with the test compound.

The test compound is not particularly restricted, and as examples there may be mentioned proteins, peptides, non-peptide compounds and artificially synthesized compounds.

The cells expressing the simian NPY Y4 receptor gene may be prepared by a protocol that is publicly known to those skilled in the art, and while there are no particular restrictions on the specific method employed, the following may be described as an example. First, a nucleic acid having the nucleotide sequence listed as SEQ ID NO: 1 or 3 or comprising a portion thereof according to the invention is cloned into an expression vector containing a suitable promoter and a suitable transcriptional control element. The vector is not particularly restricted so long as it can be used as an expression vector, and as examples there may be mentioned pcDNA3.1, pBlueBacHis2, pCI-neo, pcDNAI, pMC1neo, pXT1, pSG5, pEF1/V5-HisB, pCR2.1, pET11 and λgt11.

Next, the expression vector (recombinant vector) having the nucleic acid or gene of the invention inserted therein is transferred into host cells. The host cell is not particularly restricted so long as it is commonly used for gene expression, and may be an animal cell, insect cell, plant cell or microbe, specific examples of which include COS1, COS7, CHO, NIH/3T3, 293, Raji, CV11, C1271, MRC-5, CPAE, L-M(TK-), HeLa and Sf9. The method of transferring the recombinant vector into host cells is not particularly restricted so long as it is a publicly known method, and specifically there may be mentioned electroporation, the calcium phosphate method, the DEAE-dextran method, lipofection and the gene gun method.

The test compound is then contacted with the cells (transformant cells) expressing the NPY Y4 receptor prepared in the manner described above. The method of contact is not particularly restricted, and for example, there may be mentioned contact in a solution such as an aqueous solution or buffer solution. Binding of the test compound to receptors expressed on the cell surfaces may for example be detected with a label attached to the bound compound (for example, by radioactivity or fluorescent intensity), or determined by a binding assay using a cellular membrane fraction, which methods may be carried out by protocols which are publicly known to those skilled in the art. Detection may also be accomplished based on signal transduction in cells by binding of the test compound to the cell surface receptors, for example, using G protein activation, variations in Ca²⁺ or cAMP concentration (FLIPR (Fluorometric Imaging Plate Reader) or the like can be used), phospholipase C activation or pH variation. The expression level and activity of molecules in the signal transduction pathway which are produced by the signal transduction may also be the basis of detection. When detection is based on the expression level, there are no particular restrictions on the method of assaying the expression level, and for example, Northern blotting, real-time PCR or a DNA chip may be employed. When detection is based on the activity of a molecule in the signal transduction pathway, the activity assay method is not particularly restricted and may be selected from among suitable methods for the type of molecule to be assayed.

On the other hand, an isolated simian NPY Y4 receptor protein may also be used directly for evaluation of compounds. Spacifically, there may be employed a method wherein a test compound is contacted with a simian NPY Y4 receptor and then a change in activity of the protein resulting from the contact is detected. The method of the contact is not particularly restricted, and as specific examples there may be mentioned a method wherein contact is carried out by admixture in a buffer solution (phosphate buffer or the like) or other solution, or a method wherein the protein is immobilized on a membrane and contacted with the test compound on the membrane.

The change in activity of the protein resulting from the contact is then detected.

The method of detecting a change in activity of the protein may be appropriately set depending on the nature of the protein used, and as a specific example there may be mentioned a method based on the binding activity of NPY or a related peptide for the NPY Y4 receptor.

There are no particular restrictions on the method based on the binding activity of NPY or a related peptide, and specifically there may be mentioned a method wherein binding activity is assayed by measuring the affinity of a test compound for a membrane on which the NPY Y4 receptor protein is immobilized. The test compound used here may be labeled with a radioactive isotope etc. for easier detection. The method employed for detection of binding activity may be a method wherein a compound which binds to the NPY Y4 receptor competitively against NPY or its related peptide that has been labeled with a radioactive isotope is detected, which method requires no labeling of the test compound.

Upon detection by the compound evaluation method described above, if the binding activity of NPY or its related peptide in the presence of the test compound is a lower value than the binding activity of NPY or its related peptide in the absence of the test compound (control), the test compound is judged as inhibiting binding of the NPY Y4 receptor to its ligand or an analog thereof. Such compounds include those with activity of inducing signal transduction in cells upon binding to the receptor (agonists) and those without such activity (antagonists). Agonists have the same bioactivity as ligands for the receptor. Antagonists, on the other hand, do not have the bioactivity of ligands for the receptor while inhibiting binding of ligands to the receptor. Consequently, such agonists and antagonists are useful in pharmaceutical compositions for treatment of diseases associated with defective signal transduction via the NPY Y4 receptor.

The compound evaluation method of the invention allows screening of substances that promote or inhibit NPY Y4 receptor activity. That is, by evaluating different test compounds by the method described above it is possible to select compounds that function as agonists or antagonists. If, as a result of selection, change in the cells is suppressed compared to the change in the cells with a ligand or its analog in the absence of the test compound, then the test compound is judged to be a compound that inhibits the activity of the NPY Y4 receptor. Conversely, if the test compound augments change in the cells, then the compound is judged as being a compound that promotes the activity of the NPY Y4 receptor. Compounds selected by this screening method are expected to be useful for prevention or treatment of various conditions associated with NPY, including circulatory system disorders such as angina pectoris, acute/congestive heart failure, myocardial infarction, hypertension, kidney disease, electrolyte imbalance and vasospasm, nervous system disorders such as bulimia, depression, anxiety, convulsion, epilepsy, dementia, pain, alcoholism, drug withdrawal symptoms, circadian rhythm alteration, schizophrenia, dysmnesia, sleep disorder and cognitive impairment, metabolic disorders such as obesity, diabetes, hormone secretion imbalances, gout and fatty liver, and reproductive disorders such as infertility, premature birth and sexual dysfunction, as well as gastrointestinal diseases, respiratory diseases, inflammatory conditions, hypercholesterolemia, hyperlipidemia, arteriosclerosis and glaucoma.

The compound evaluation method of the invention as described above also allows evaluation of ligands used for PET (Positron Emission Tomography). PET is a non-invasive method for observing biological function by labeling a substance found in the body such as water, oxygen, glucose or an amino acid, or a drug (ligand for a target receptor, for example) with a radioactive substance and administering it to the body, and the method is used for research and in clinical practice. PET allows function-specific imaging dependent on the ligand used as the tracer, and development of new tracers is indispensable for elucidation of unknown biological functions and diagnosis of disease. In the compound evaluation method of the present invention, the PET ligand candidate substance may be used as the test compound to allow *in vitro* evaluation of the substance.

### (2) Probe used for hybridization

The NPY Y4 receptor gene can be detected by using a nucleic acid consisting of all or a portion of the nucleotide sequence listed as SEQ ID NO: 1 or 3 of the invention as a hybridization probe. The detection is not limited to detection of the NPY Y4 receptor gene in a specimen of biological tissue or cells, but may also be applied for cloning of the NPY Y4 receptor gene or a gene having high homology to the gene. Also, the nucleic acid may be used as a probe for identification of gene expression distribution by examining gene expression in different tissues.

When the nucleic acid is used as a probe, there are no particular restrictions on the method of hybridization, and as examples there may be mentioned Southern hybridization, Northern hybridization, colony hybridization, dot hybridization, fluorescence in situ hybridization (FISH), in situ hybridization (ISH) and the DNA chip method.

When the nucleic acid is used as a probe for hybridization, a nucleic acid with a length of at least 20 contiguous nucleotides is required, and the length is preferably at least 40 nucleotides, more preferably at least 60 nucleotides and most preferably at least 80 nucleotides. The probe may also be labeled allow detection if necessary. Specifically, it may be labeled with a radioactive isotope such as ³²P, ¹⁴C, ¹²⁵I, ³H or ³⁵S, or it may be labeled with biotin, a fluorescent dye, an enzyme, gold colloid or the like.

The conditions for hybridization when the nucleic acid is used as a probe for hybridization may be selected as appropriate by a person skilled in the art depending on the length of the probe, and the type of gene which is the target of hybridization. It may be carried out, for example, with reference to Molecular Cloning: A Laboratory Manual, 2nd Edition, Cold Spring Harbor (1989), 9.47-9.62 and 11.45-11.61. More specifically, there may be mentioned conditions wherein hybridization is conducted at about 45°C, 6.0 x SSC and followed by rinsing at 50°C, 2.0 x SSC. For selection of stringency, the salt concentration for the rinsing step may be selected from about 2.0 x SSC, 50°C for low stringency to about 0.2 x SSC, 50°C for high stringency. The temperature for the rinsing step may be from room temperature (about 22°C) for low stringency conditions to about 65°C for high stringency conditions.

### (3) Primers used for PCR

The method of detecting the NPY Y4 gene may be the Polymerase Chain Reaction (PCR) using portions of the nucleotide sequences listed as SEQ ID NO: 1 or 3 of the invention as primers.

The lengths of the primers used may be appropriately selected based on their nucleotide sequences and the nucleotide sequence of a gene to be isolated, but generally, they will be preferably 10-60, more preferably 15-30, contiguous nucleotides.

### Examples

The present invention will now be explained in greater detail through examples, with the understanding that these examples are in no way limitative on the invention.

### (Example 1: Isolation of rhesus monkey Y4 receptor gene)

The two oligo DNA primers hY4-4FBamHI (SEQ ID NO: 5) and hY4-1323RNotI2 (SEQ ID NO: 6) were designed based on the nucleotide sequence of the 5' and 3' untranslated regions of the human Y4 receptor gene (GenBank Accession No. U35232). These primers were used with rhesus monkey genomic DNA (Clontech) as a template for a PCR reaction of 40 cycles, where one cycle was 94°C for 10 seconds (denaturation)/ 55°C for 1 minute (annealing)/ 68°C for 2 minutes (elongation). The obtained PCR amplification product was digested with the restriction endonucleases BamHI and NotI, and cloned into a pEF1/V5-HisB plasmid vector (Invitrogen). Six clones were randomly selected from among clones obtained by six independent PCR reactions, and the 10 primers T7, BGHreverse, Y41, Y42, Y43, Y44, Y45, Y46, Y47 and Y48 (SEQ ID NO: 7-16) were used for determination of the nucleotide sequences by the dye terminator method. The nucleotide sequences of the two rhesus monkey Y4 receptor genes listed as SEQ ID NO: 1 and 3 were elucidated by comparison of the nucleotide sequences of the six clones.

The sequences listed as SEQ ID NO: 1 and 3 each have an open reading frame of 1128 nucleotides (positions 30-1157). The amino acid sequences (375 residues) predicted from these open reading frames are listed as SEQ ID NO: 2 and 4. SEQ ID NO: 1 and 3 differ by one nucleotide in the coding region (position 1111), and the predicted amino acid sequences of SEQ ID NO: 2 and 4 differ only in the 361st amino acid.

Upon comparing the sequences listed as SEQ ID NO: 2 and 4 with the human Y4 receptor protein amino acid sequence (GenPept Accession No. NP_005963), very high homologies of 96% (359 of 375 residues are identical) and 95% (358 of 375 residues are identical) were found, respectively, suggesting that the nucleotide sequences listed as SEQ ID NO: 1 and 3 code for rhesus monkey Y4 receptor proteins.

The nucleotide sequences of the primers used were as follows.
hY4-4FBamHI: ATCGGATCCG TCATCCCTCA AGTGTATCAC TTAGTTC (SEQ ID NO: 5)
hY4-1323RNotI: CATGCGGCCG CAAACAGCTC TTGCCAGCCT ATCTGG (SEQ ID NO: 6)
T7: TAATACGACT CACTATAGGG (SEQ ID NO: 7)
BGHreverse: GGAGCTGACA CGGAAGAT (SEQ ID NO: 8)
Y41: GGGACATGGC AGGGAGAAGA (SEQ ID NO: 9)
Y42: GCTGTTGAAC ACATGCAGAG (SEQ ID NO: 10)
Y43: TCTTGTGGAA GACATTCTCC (SEQ ID NO: 11)
Y44: AGAAGGCCAG GTTGGCGATA (SEQ ID NO: 12)
Y45: GATGGTCTTC ATCGTCACTT (SEQ ID NO: 13)
Y46: CAGCTCATCA TCAACCCAAC (SEQ ID NO: 14)
Y47: ATCAAGGCCC TGGTGCTGAC TTGC (SEQ ID NO: 15)
Y48: TTATGCACGC ATCTACCGGC (SEQ ID NO: 16)

### (Example 2: Rhesus monkey Y4 receptor expression and [¹²⁵I]human PP binding assay using COS-7 cells)

COS-7 cells (American Type Culture Collection) were seeded in a 24-well culturing plate at a density of 5 x 10⁴ cells/well, and after 24 hours, they were transfected with a pEF1/V5-HisB plasmid vector containing the nucleotide sequence of SEQ ID NO: 1 or 3 obtained in Example 1 or the same vector without the insert (mock), using Efectene (Qiagen). After culturing for 48 hours, incubation was performed at 37°C for 30 minutes in 240 µl/well of reaction buffer (Hanks' balanced salt solution containing 20 mM HEPES and 0.2% bovine serum albumin; pH 7.4) to which 50 pM [¹²⁵I]human PP (Perkin-Elmer) had been added. After rinsing with ice-cooled reaction buffer, the cells were lysed and recovered with 2M NaOH and the radioactivity was measured with a γ-counter (Packard, Cobra).

Non-specific binding was also measured by adding 1 µM human PP to the reaction solution. As shown in Table 1, the COS-7 cells transfected with the expression vector containing the nucleotide sequence of SEQ ID NO: 1 or 3 exhibited specific binding to [¹²⁵I]human PP, whereas no specific binding was observed with the cells transfected with the vector without the insert (mock).

This example demonstrated that proteins encoded by the nucleotide sequences listed as SEQ ID NO: 1 and 3 are capable of PP binding, which is a characteristic of the Y4 receptor.

**[Table 1]**

| Expression vector | Total binding (cpm) | Non-specific binding (cpm) | Specific binding (cpm) |
|---|---|---|---|
| SEQ ID NO: 1 | 1297 | 59 | 1238 |
| SEQ ID NO: 3 | 1456 | 62 | 1394 |
| Mock | 70 | 68 | 2 |

### (Example 3: Receptor binding assay using rhesus monkey Y4 receptor-expressing COS-7 cell membrane fractions)

COS-7 cells were transfected with a pEF1/V5-HisB plasmid vector containing the nucleotide sequence of SEQ ID NO: 1 or 3, using LipofectAmine Plus (Invitrogen). After 48 hours, the cells were recovered, and disrupted in 10 mM MOPS buffer (pH 7.4) containing 20% sucrose, 154 mM NaCl, 10 mM KCl and 0.8 mM CaCl₂, using a Polytron homogenizer (Kinematica) while cooling on ice. The obtained homogenate was centrifuged at 1,000 x g, 4°C for 15 minutes, the supernatant was centrifuged at 90,000 x g for 50 minutes, and the precipitate was collected. It was then suspended in 5 mM HEPES/Tris buffer (pH 7.4) and then centrifuged again at 90,000 x g for 50 minutes, after which the obtained precipitate was collected as the membrane sample.

The membrane fraction obtained in this manner was incubated for 2 hours at 25°C together with 31 pM [¹²⁵I]human PP in 25 mM Tris buffer (pH 7.4) containing 10 mM MgCl₂, 1 mM phenylmethylsulfonyl fluoride, 0.1% bacitracin and 0.5% bovine serum albumin, and then filtered through a polyethyleneimine-treated UniFilter-96 GF/C (Perkin-Elmer). After rinsing with 5 mM Tris buffer (pH 7.4) containing 0.3% bovine serum albumin and drying, MicroScint0 (Perkin-Elmer) was added, and a TopCount HTS (Packard) was used to measure the residual radioactivity on the filter. The non-specific binding was measured in the presence of 1 µM human PP.

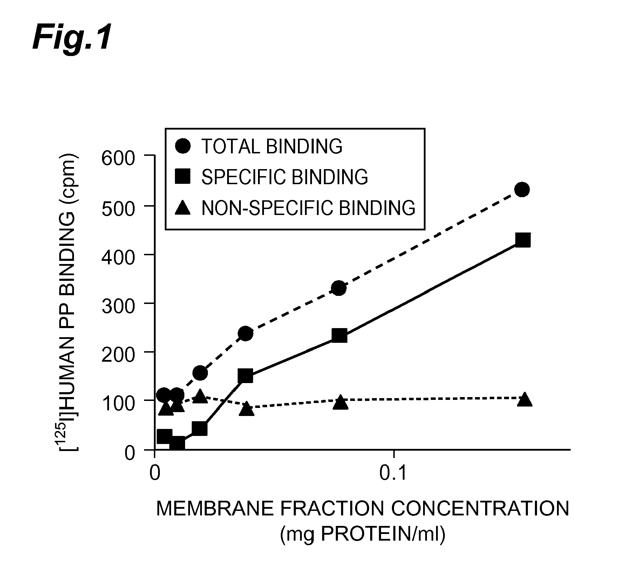

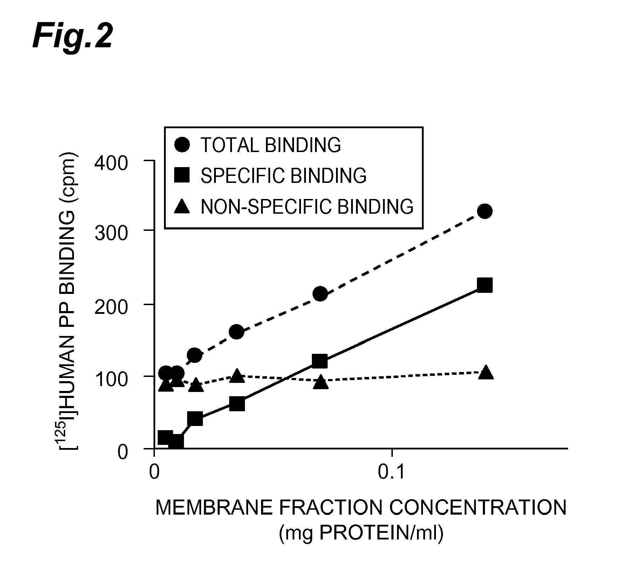

As shown in Fig. 1 and Fig. 2, both of the membrane samples of cells transfected with the sequences of SEQ ID NO: 1 and 3 exhibited specific binding of [¹²⁵I]human PP in a manner dependent on membrane fraction concentration.

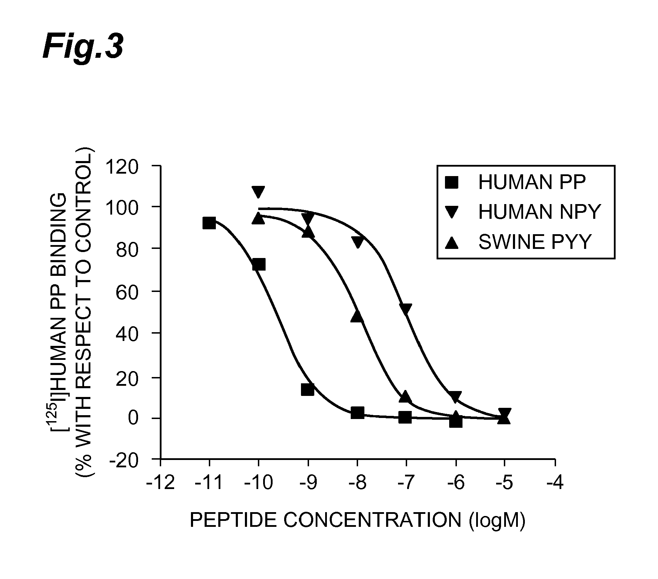

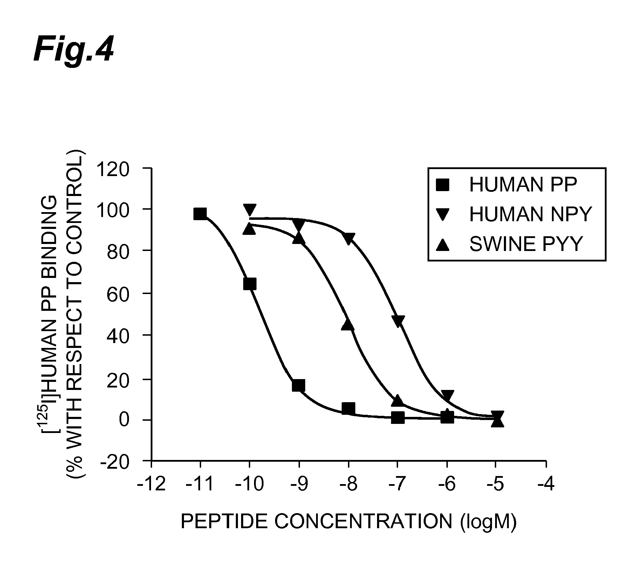

Also, with the membrane fraction concentration set to 0.155 and 0.14 mg protein/ml and the [¹²⁵I]human PP concentration set to 59 pM, human PP, human NPY and swine PYY were added to the reaction solution at different concentrations, and a Prism Version 4.00 (GraphPad) was used to determine the 50% inhibitory concentration (IC₅₀ value) from the inhibition curve for each peptide against specific [¹²⁵I]human PP binding (Figs. 3 and 4). The IC₅₀ value for each peptide is shown in Table 2, indicating that proteins encoded by the nucleotide sequences SEQ ID NO: 1 and 3 exhibit the same properties as the known Y4 receptor, which has higher affinity for PP than for NPY or PYY [The Journal Biological Chemistry, Vol. 270, 26762 (1995); The Journal Biological Chemistry, Vol. 270, 29123 (1995); FEBS Letters, Vol. 381, 58 (1996); Proceedings of the National Academy of Sciences of the United States of America, Vol. 93, 4661 (1995); Proceedings of the National Academy of Sciences of the United States of America, Vol. 93, 5111 (1995)].

**[Table 2]**

| Expression vector | IC₅₀ (nM) | | |
|---|---|---|---|
| | Human PP | Human NPY | Swine PYY |
| SEQ ID NO: 1 | 0.24 | 100 | 10 |
| SEQ ID NO: 3 | 0.17 | 97 | 9.9 |

### (Example 4: Measurement of intracellular calcium ion concentration increase by PP in CHO-K1 cells coexpressing rhesus monkey Y4 receptor and Gα₁₅ protein)

CHO-K1 cells (Aurora Biosciences, CHO.Gα15-blast.3xNFAT-blaX-zeo.S1.C3-21) stably expressing Gα₁₅, which is a promiscuous G protein α subunit, were transfected with a pEF1/V5-HisB plasmid vector containing the nucleotide sequence of SEQ ID NO: 1 or 3 or the same vector without the insert (mock), using Efectene (Qiagen), and geneticin (Invitrogen)-resistant cells were selected. The obtained cells were seeded in a ViewPlate-96 (Perkin-Elmer) at 2 x 10⁴ cells/well, and after culturing for 48 hours, incubation was performed at 37°C for one hour in assay buffer (Hanks' balanced salt solution containing 20 mM HEPES, 2.5 mM probenecid and 0.5% bovine serum albumin; pH 7.4) containing 2 µM Fluo-4, AM (Molecular Probes) and 1% fetal calf serum, for loading of the calcium ion indicator Fluo-4 into the cells. After rinsing 4 times with assay buffer, the cells were placed in an FLIPR (Molecular Devices) and the change in intracellular calcium ion concentration with addition of different concentrations of human PP was measured based on the change in fluorescent intensity.

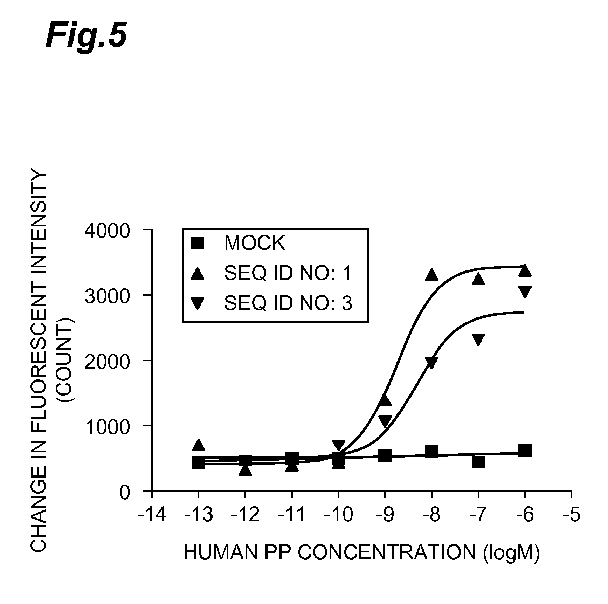

As shown in Fig. 5, an increase in fluorescent intensity dependent on human PP concentration was observed with the cells transfected with the sequence of SEQ ID NO: 1 or 3, indicating that PP raises intracellular calcium ion concentration in these cells. Based on the concentration response curve, the EC₅₀ values were calculated as 1.8 nM (SEQ ID NO: 1) and 4.8 nM (SEQ ID NO: 3). On the other hand, the cells transfected with the vector without the insert (mock) showed no such PP addition-dependent increase in fluorescent intensity.

This test demonstrated that proteins encoded by the nucleotide sequences listed as SEQ ID NO: 1 and 3 can be coupled to G protein upon interaction with PP, inducing intracellular signal transduction.

### Industrial Applicability

According to the nucleic acid, protein and compound evaluation method of the invention, it is possible to obtain an NPY Y4 receptor gene and protein of a non-human primate, and compound evaluation and screening can be carried out using the gene or protein. Thus, therapeutic and diagnostic agents can be evaluated and developed using the gene or protein. A major contribution is therefore provided for increased efficiency of research and development of human drugs.

## Claims

1. An isolated nucleic acid consisting of the nucleotide sequence listed as SEQ ID NO: 1 or 3.

2. An isolated nucleic acid coding for a protein consisting of the amino acid sequence listed as SEQ ID NO: 2 or 4.

3. An isolated simian nucleic acid which hybridizes under stringent conditions with a nucleic acid consisting of the nucleotide sequence listed as SEQ ID NO: 1 or 3 or a nucleotide sequence complementary thereto and which codes for a protein having NPY Y4 receptor activity.

4. A simian NPY Y4 receptor gene consisting of the nucleic acid according to any one of claims 1 to 3.

5. A recombinant vector containing the simian NPY Y4 receptor gene according to claim 4.

6. A transformed cell containing the recombinant vector according to claim 5.

7. An isolated protein consisting of the amino acid sequence listed as SEQ ID NO: 2 or 4.

8. An isolated protein consisting of the amino acid sequence listed as SEQ ID NO: 2 or 4 with the substitution, deletion, addition or insertion of one or more amino acids, and having NPY Y4 receptor activity.

9. A compound evaluation method comprising:
a step of transferring a simian NPY Y4 receptor gene into a cell to prepare a cell expressing the receptor,
a step of contacting a test compound with the cell, and
a step of detecting specific binding of the test compound to the receptor.

10. A compound evaluation method comprising:
a step of transferring a simian NPY Y4 receptor gene into a cell to prepare a cell expressing the receptor,
a step of contacting a test compound with the cell,
a step of assaying the activity of an intracellular signal transducer produced by the contact, and
a step of comparing the activity with the activity of the intracellular signal transducer without contact with the test compound.

11. A compound evaluation method comprising:
a step of contacting a test compound with a simian protein having NPY Y4 receptor activity, and
a step of detecting a change in activity of the protein caused by the contact.

12. The compound evaluation method according to any one of claims 9 to 11, wherein the simian is a rhesus monkey.


SEQUENCE LISTING

<110>  BANYU PHARMACEUTICAL CO., LTD

<120>  rhesus NPY Y4 receptor and evaluation method of a compound

<130>  0328

<160>  16

<170>  PatentIn version 3.1

<210>  1
<211>  1267
<212>  DNA
<213>  rhesus monkey

<400>  1
gagtgtcctg gaatcttttc acatccacca tgaacacctc tcacctcctg gccttgcagc      60

tgccaaaatc cctacagggt gaaaacagaa gcaagcccct gggcatccca tacaacttct     120

ctgacccttg ccaggattct gtggacatga tggtcttcat cgtcacttcc tacagcattg     180

agactgtcgt gggggtcctg ggtaacctct gcctgatgtg tgtaactgtg aggcagaagg     240

agaaaaccaa tgtgaccaac ctgcttatca ccaacctggc cttctctgac ttcctcatgt     300

gcctcctctg ccagccgctg accgccatct ataccatcat ggattactgg atcttcggag     360

agaccctctg caagatgtcg gccttcatcc agtgtgtgtc ggtgacggtc tccatcctct     420

cgctcgtcct tgtggccctg gagaggcatc agctcatcat caacccaaca ggctggaagc     480

ccagcatctc tcaggcttac ctggggattg tgctcatctg ggtcattgcc tgtgtcctct     540

ccctgccctt cctggccaat agcatcctgg agaatgtctt ccacaagaac cactccaagg     600

ctctggagtt cctggcggat aaggtggtct gtactgagtc ctggccactg gctcaccacc     660

gcaccatcta taccaccttc ctcctcctct tccagtactg cctcccactg ggcttcatcc     720

tggtctgtta tgcacgcatc taccggcgcc tgcagaggca ggggcgtgtg ttccacaagg     780

gcacctacag cttgcgagct ggacagatga agcaggtcaa tgtggtgctg gtggcgatgg     840

tggtggcctt cgctgtgctc tggctgcctc tgcatgtgtt caacagcctg gaggactggc     900

accatgaggc catccccatc tgccatggga acctcatctt cttggtgtgc cacttgcttg     960

ccatggcctc cacctgtgtc aacccattca tctacggctt tctcaacacc aacttcaaga    1020

aggaaatcaa ggccctggtg ctgacgtgcc agcagagtcc ccccctggag gagttggagc    1080

atctacccct gtccacagta cacacggaag tctccaaagt gtccctgagg ctaaatggca    1140

ggtccaatcc catttaatca ggtctagggc ttctccctgc catgtccctt gccaggctct    1200

ttcacttagc gaagtgggca cactgcaagc tggggtggca ccccagcatt cctggctttc    1260

tggggcc                                                              1267

<210>  2
<211>  375
<212>  PRT
<213>  rhesus monkey

<400>  2

Met Asn Thr Ser His Leu Leu Ala Leu Gln Leu Pro Lys Ser Leu Gln
1               5                   10                  15

Gly Glu Asn Arg Ser Lys Pro Leu Gly Ile Pro Tyr Asn Phe Ser Asp
            20                  25                  30

Pro Cys Gln Asp Ser Val Asp Met Met Val Phe Ile Val Thr Ser Tyr
        35                  40                  45

Ser Ile Glu Thr Val Val Gly Val Leu Gly Asn Leu Cys Leu Met Cys
    50                  55                  60

Val Thr Val Arg Gln Lys Glu Lys Thr Asn Val Thr Asn Leu Leu Ile
65                  70                  75                  80

Thr Asn Leu Ala Phe Ser Asp Phe Leu Met Cys Leu Leu Cys Gln Pro
                85                  90                  95

Leu Thr Ala Ile Tyr Thr Ile Met Asp Tyr Trp Ile Phe Gly Glu Thr
            100                 105                 110

Leu Cys Lys Met Ser Ala Phe Ile Gln Cys Val Ser Val Thr Val Ser
        115                 120                 125

Ile Leu Ser Leu Val Leu Val Ala Leu Glu Arg His Gln Leu Ile Ile
    130                 135                 140

Asn Pro Thr Gly Trp Lys Pro Ser Ile Ser Gln Ala Tyr Leu Gly Ile
145                 150                 155                 160

Val Leu Ile Trp Val Ile Ala Cys Val Leu Ser Leu Pro Phe Leu Ala
                165                 170                 175

Asn Ser Ile Leu Glu Asn Val Phe His Lys Asn His Ser Lys Ala Leu
            180                 185                 190

Glu Phe Leu Ala Asp Lys Val Val Cys Thr Glu Ser Trp Pro Leu Ala
        195                 200                 205

His His Arg Thr Ile Tyr Thr Thr Phe Leu Leu Leu Phe Gln Tyr Cys
    210                 215                 220

Leu Pro Leu Gly Phe Ile Leu Val Cys Tyr Ala Arg Ile Tyr Arg Arg
225                 230                 235                 240

Leu Gln Arg Gln Gly Arg Val Phe His Lys Gly Thr Tyr Ser Leu Arg
                245                 250                 255

Ala Gly Gln Met Lys Gln Val Asn Val Val Leu Val Ala Met Val Val
            260                 265                 270

Ala Phe Ala Val Leu Trp Leu Pro Leu His Val Phe Asn Ser Leu Glu
        275                 280                 285

Asp Trp His His Glu Ala Ile Pro Ile Cys His Gly Asn Leu Ile Phe
    290                 295                 300

Leu Val Cys His Leu Leu Ala Met Ala Ser Thr Cys Val Asn Pro Phe
305                 310                 315                 320

Ile Tyr Gly Phe Leu Asn Thr Asn Phe Lys Lys Glu Ile Lys Ala Leu
                325                 330                 335

Val Leu Thr Cys Gln Gln Ser Pro Pro Leu Glu Glu Leu Glu His Leu
            340                 345                 350

Pro Leu Ser Thr Val His Thr Glu Val Ser Lys Val Ser Leu Arg Leu
        355                 360                 365

Asn Gly Arg Ser Asn Pro Ile
    370                 375

<210>  3
<211>  1267
<212>  DNA
<213>  rhesus monkey

<400>  3
gagtgtcctg gaatcttttc acatccacca tgaacacctc tcacctcctg gccttgcagc      60

tgccaaaatc cctacagggt gaaaacagaa gcaagcccct gggcatccca tacaacttct     120

ctgacccttg ccaggattct gtggacatga tggtcttcat cgtcacttcc tacagcattg     180

agactgtcgt gggggtcctg ggtaacctct gcctgatgtg tgtaactgtg aggcagaagg     240

agaaaaccaa tgtgaccaac ctgcttatca ccaacctggc cttctctgac ttcctcatgt     300

gcctcctctg ccagccgctg accgccatct ataccatcat ggattactgg atcttcggag     360

agaccctctg caagatgtcg gccttcatcc agtgtgtgtc ggtgacggtc tccatcctct     420

cgctcgtcct tgtggccctg gagaggcatc agctcatcat caacccaaca ggctggaagc     480

ccagcatctc tcaggcttac ctggggattg tgctcatctg ggtcattgcc tgtgtcctct     540

ccctgccctt cctggccaat agcatcctgg agaatgtctt ccacaagaac cactccaagg     600

ctctggagtt cctggcggat aaggtggtct gtactgagtc ctggccactg gctcaccacc     660

gcaccatcta taccaccttc ctcctcctct tccagtactg cctcccactg ggcttcatcc     720

tggtctgtta tgcacgcatc taccggcgcc tgcagaggca ggggcgtgtg ttccacaagg     780

gcacctacag cttgcgagct ggacagatga agcaggtcaa tgtggtgctg gtggcgatgg     840

tggtggcctt cgctgtgctc tggctgcctc tgcatgtgtt caacagcctg gaggactggc     900

accatgaggc catccccatc tgccatggga acctcatctt cttggtgtgc cacttgcttg     960

ccatggcctc cacctgtgtc aacccattca tctacggctt tctcaacacc aacttcaaga    1020

aggaaatcaa ggccctggtg ctgacgtgcc agcagagtcc ccccctggag gagttggagc    1080

atctacccct gtccacagta cacacggaag cctccaaagt gtccctgagg ctaaatggca 1140

ggtccaatcc catttaatca ggtctagggc ttctccctgc catgtccctt gccaggctct 1200

ttcacttagc gaagtgggca cactgcaagc tggggtggca ccccagcatt cctggctttc 1260

tgggggc 1267

<210> 4
<211> 375
<212> PRT
<213> rhesus monkey

<400> 4

Met Asn Thr Ser His Leu Leu Ala Leu Gln Leu Pro Lys Ser Leu Gln
1               5                   10                  15

Gly Glu Asn Arg Ser Lys Pro Leu Gly Ile Pro Tyr Asn Phe Ser Asp
            20                  25                  30

Pro Cys Gln Asp Ser Val Asp Met Met Val Phe Ile Val Thr Ser Tyr
        35                  40                  45

Ser Ile Glu Thr Val Val Gly Val Leu Gly Asn Leu Cys Leu Met Cys
    50                  55                  60

Val Thr Val Arg Gln Lys Glu Lys Thr Asn Val Thr Asn Leu Leu Ile
65                  70                  75                  80

Thr Asn Leu Ala Phe Ser Asp Phe Leu Met Cys Leu Leu Cys Gln Pro
                85                  90                  95

Leu Thr Ala Ile Tyr Thr Ile Met Asp Tyr Trp Ile Phe Gly Glu Thr
            100                 105                 110

Leu Cys Lys Met Ser Ala Phe Ile Gln Cys Val Ser Val Thr Val Ser
        115                 120                 125

Ile Leu Ser Leu Val Leu Val Ala Leu Glu Arg His Gln Leu Ile Ile
    130                 135                 140

Asn Pro Thr Gly Trp Lys Pro Ser Ile Ser Gln Ala Tyr Leu Gly Ile
145                 150                 155                 160

Val Leu Ile Trp Val Ile Ala Cys Val Leu Ser Leu Pro Phe Leu Ala
                165                 170                 175

Asn Ser Ile Leu Glu Asn Val Phe His Lys Asn His Ser Lys Ala Leu
            180                 185                 190

Glu Phe Leu Ala Asp Lys Val Val Cys Thr Glu Ser Trp Pro Leu Ala
        195                 200                 205

His His Arg Thr Ile Tyr Thr Thr Phe Leu Leu Leu Phe Gln Tyr Cys
    210                 215                 220

Leu Pro Leu Gly Phe Ile Leu Val Cys Tyr Ala Arg Ile Tyr Arg Arg
225                 230                 235                 240

Leu Gln Arg Gln Gly Arg Val Phe His Lys Gly Thr Tyr Ser Leu Arg
                245                 250                 255

Ala Gly Gln Met Lys Gln Val Asn Val Val Leu Val Ala Met Val Val
            260                 265                 270

Ala Phe Ala Val Leu Trp Leu Pro Leu His Val Phe Asn Ser Leu Glu
        275                 280                 285

Asp Trp His His Glu Ala Ile Pro Ile Cys His Gly Asn Leu Ile Phe
    290                 295                 300

Leu Val Cys His Leu Leu Ala Met Ala Ser Thr Cys Val Asn Pro Phe
305                 310                 315                 320

Ile Tyr Gly Phe Leu Asn Thr Asn Phe Lys Lys Glu Ile Lys Ala Leu
                325                 330                 335

Val Leu Thr Cys Gln Gln Ser Pro Pro Leu Glu Glu Leu Glu His Leu
            340                 345                 350

Pro Leu Ser Thr Val His Thr Glu Ala Ser Lys Val Ser Leu Arg Leu
        355                 360                 365

Asn Gly Arg Ser Asn Pro Ile
    370                 375

<210>  5
<211>  37
<212>  DNA
<213>  Artificial sequence

<220>
<223>  synthetic polynucleotide

<400>  5
atcggatccg tcatccctca agtgtatcac ttagttc                              37

<210>  6
<211>  36
<212>  DNA
<213>  Artificial sequence

<220>
<223>  synthetic polynucleotide

<400>  6
catgcggccg caaacagctc ttgccagcct atctgg                               36

<210>  7

<211>  20
<212>  DNA
<213>  Artificial sequence

<220>
<223>  synthetic polynucleotide

<400>  7
taatacgact cactataggg                                                    20


<210>  8
<211>  18
<212>  DNA
<213>  Artificial sequence

<220>
<223>  synthetic polynucleotide

<400>  8
ggagctgaca cggaagat                                                      18


<210>  9
<211>  20
<212>  DNA
<213>  Artificial sequence

<220>
<223>  synthetic polynucleotide

<400>  9
gggacatggc agggagaaga                                                    20


<210>  10
<211>  20
<212>  DNA
<213>  Artificial sequence

<220>
<223>  synthetic polynucleotide

<400>  10
gctgttgaac acatgcagag                                                    20


<210>  11
<211>  20
<212>  DNA
<213>  Artificial sequence

<220>
<223>  synthetic polynucleotide

<400>  11
tcttgtggaa gacattctcc                                                    20


<210>  12
<211>  20
<212>  DNA
<213>  Artificial sequence

<220>
<223>  synthetic polynucleotide

<400>  12
agaaggccag gttggcgata                                                    20

<210>  13
<211>  20
<212>  DNA
<213>  Artificial sequence

<220>
<223>  synthetic polynucleotide

<400>  13
gatggtcttc atcgtcactt                                                    20


<210>  14
<211>  20
<212>  DNA
<213>  Artificial sequence

<220>
<223>  synthetic polyucleotide

<400>  14
cagctcatca tcaacccaac                                                    20


<210>  15
<211>  24
<212>  DNA
<213>  Artificial sequence

<220>
<223>  synthetic polynucleotide

<400>  15
atcaaggccc tggtgctgac ttgc                                               24


<210>  16
<211>  20
<212>  DNA
<213>  Artificial sequence

<220>
<223>  synthetic polynucleotide

<400>  16
ttatgcacgc atctaccggc                                                    20